(19) **Europäisches Patentamt**
**European Patent Office**
**Office européen des brevets**

(11) **EP 1 723 094 B1**

(12) **EUROPÄISCHE PATENTSCHRIFT**

(45) Veröffentlichungstag und Bekanntmachung des Hinweises auf die Patenterteilung:
**11.06.2014 Patentblatt 2014/24**

(21) Anmeldenummer: **05715282.9**

(22) Anmeldetag: **10.02.2005**

(51) Int Cl.:
***C07C 47/058*** (2006.01)

(86) Internationale Anmeldenummer:
**PCT/EP2005/001318**

(87) Internationale Veröffentlichungsnummer:
**WO 2005/077877 (25.08.2005 Gazette 2005/34)**

(54) **VERFAHREN ZUR HERSTELLUNG EINER HOCHKONZENTRIERTEN FORMALDEHYDLÖSUNG**

METHOD FOR PRODUCING HIGHLY CONCENTRATED FORMALDEHYDE SOLUTION

PROCEDE POUR LA PREPARATION D' UNE SOLUTION HAUTEMENT CONCENTREE DE FORMALDEHYDE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **11.02.2004 DE 102004006649**

(43) Veröffentlichungstag der Anmeldung:
**22.11.2006 Patentblatt 2006/47**

(73) Patentinhaber: **BASF SE**
**67056 Ludwigshafen (DE)**

(72) Erfinder:
• **RUMPF, Bernd**
**68766 Hockenheim (DE)**

• **STROEFER, Eckhard**
**68163 Mannheim (DE)**
• **LANG, Ortmund**
**66909 Quirnbach (DE)**
• **LANG, Neven**
**68163 Mannheim (DE)**

(56) Entgegenhaltungen:
**WO-A-03/040075**

• **CASPER C ET AL: "DAS MEHRPHASENWENDELROHR ALS HOCHLEISTUNGSSTOFFAUSTAUSCHER" CHEMIEINGENIEURTECHNIK, WILEY VCH., WEINHEIM, DE, Bd. 68, 1996, Seiten 706-710, XP000197907 ISSN: 0009-286X**

EP 1 723 094 B1

**Beschreibung**

Die Erfindung betrifft ein Verfahren zur Herstellung einer hochkonzentrierten Formaldehydlösung durch Abtrennung von Wasser aus einer niedriger konzentrierten Formaldehydlösung.

Formaldehyd ist eine wichtige Industriechemikalie und wird zur Herstellung zahlreicher Industrieprodukte und Verbrauchsartikel eingesetzt. In über 50 Industriezweigen wird derzeit Formaldehyd verwendet, im Wesentlichen in Form von wässrigen Lösungen oder Formaldehyd enthaltenden Kunstharzen. Kommerziell erhältliche, wässrige Formaldehydlösungen weisen Gesamtkonzentrationen von 20 bis 55 Gew.-% Formaldehyd in Form von monomerem Formaldehyd, Methylenglykol und Polyoxymethylenglykolen auf. Somit wird in industriell angewendeten Synthesen, die unter Einsatz von wässrigen Formaldehydlösungen verlaufen, zusammen mit dem Formaldehyd eine große Menge Wasser eingetragen, die in der Synthese im Allgemeinen nicht benötigt wird. Diese hohe Wasserlast bestimmt die Größe der Reaktoren, deren Peripherie sowie die Aufarbeitung der Produkte. Des Weiteren muss das überschüssige Wasser als Abwasser behandelt und entsorgt werden. Gegebenenfalls ist es erforderlich, das Wasser unter erheblichem Energieeinsatz thermisch abzutrennen. Somit ist es wünschenswert, die Wasserlast in Synthesen, die den Einsatz wässriger Formaldehydlösungen erfordern, zu verringern, indem möglichst hochkonzentrierte wässrige Formaldehydlösungen eingesetzt werden.

Die Herstellung und Verwendung solcher hochkonzentrierter wässriger Formaldehydlösungen ist jedoch problematisch, da bei höher konzentrierten Lösungen, insbesondere bei niedrigen Temperaturen, Feststoffe ausfallen. Wässrige Formaldehydlösungen mit mehr als 30 Gew.-% Formaldehyd werden bei einer Lagerung bei Raumtemperatur bereits trüb, da höhere Polyoxymethylenglykole ($HO(CH_2O)_nH$; $n \geq 8$) gebildet werden, die ausfallen. (Ullmann'2 Encyclopedia of Industrial Chemistry, Edition, 2000 electronic release, Formaldehyde; chapter 2 (physical properties), 2.2 (aqueous solutions), Seite 2, dritter Absatz). Bei höheren Temperaturen nimmt zwar die Löslichkeit der in der wässrigen Formaldehydlösung enthaltenen Produkte zu, jedoch erfolgt die unerwünschte Bildung von Ameisensäure durch Cannizzaro-Reaktion. Daher haben beispielsweise durch Destillation bei höheren Temperaturen und Drücken erzeugte hochkonzentrierte Formaldehydlösungen hohe Ameisensäuregehalte und sind somit durch niedrige pH-Werte gekennzeichnet.

In DE-A 102 38 248 wird beschrieben, dass für den Feststoffausfall in hochkonzentrierten wässrigen Formaldehydlösungen die mittlere Kettenlänge der Polyoxymethylenglykole, die mit der mittleren Molmasse derselben korreliert, maßgeblich ist. In der genannten Patentanmeldung werden wässrige Formaldehydlösungen beschrieben, enthaltend Formaldehyd in Form von monomerem Formaldehyd, Methylenglykol und Polyoxymethylenglykolen in einer Gesamtkonzentration x von $\geq$ 65 Gew.-%, die dadurch gekennzeichnet, sind, dass die mittlere Molmasse $\overline{M}$ der Polyoxymethylenglykole in Abhängigkeit von der Formaldehydkonzentration gleich oder kleiner den gemäß Formel I erhaltenen Werten ist:

$$\left(\frac{\overline{M}}{g/mol}\right) = 48 + 6{,}589 \cdot 10^{-1} \cdot \left(\frac{x}{Gew.-\%}\right) + 4{,}725 \cdot 10^{-2} \cdot \left(\frac{x}{Gew.-\%}\right)^2 - 3{,}434 \cdot 10^{-3} \cdot \left(\frac{x}{Gew.-\%}\right)^3$$

$$+ 9{,}625 \cdot 10^{-5} \cdot \left(\frac{x}{Gew.-\%}\right)^4 - 1{,}172 \cdot 10^{-6} \cdot \left(\frac{x}{Gew.-\%}\right)^5 + 5{,}357 \cdot 10^{-9} \cdot \left(\frac{x}{Gew.-\%}\right)^6$$

(I).

Darin bedeuten:

$\overline{M}$  mittlere Molmasse

x  Gesamtkonzentration an Formaldehyd in Form von monomerem Formaldehyd, Methylenglycol und Polyoxymethylenglycolen in Gew.-% (Formaldehydgesamtkonzentration).

Die beschriebenen wässrigen Formaldehydlösungen zeichnen sich bevorzugt dadurch aus, dass bei Temperaturen von im Allgemeinen -5 bis 180°C, bevorzugt 10 bis 100°C, besonders bevorzugt von Raumtemperatur bis 50°C - also bei Temperaturen, bei denen üblicherweise die Reaktionen mit Formaldehyd durchgeführt werden - über einen Zeitraum von mindestens 1 min, bevorzugt mindestens 5 min, besonders bevorzugt mindestens 1 h, kein Feststoffausfall auftritt. Insbesondere ist keine Alterung bei erhöhten Temperaturen nötig. Die Erhöhung der Temperatur ist im Allgemeinen sogar unerwünscht.

Somit sind die erfindungsgemäßen wässrigen Formaldehydlösungen überall dort einsetzbar, wo Reaktionen mit geeigneten Verbindungen in dem genannten Zeitraum ablaufen.

Die Herstellung der beschriebenen wässrigen Formaldehydlösungen erfolgt durch Entzug von Wasser oder einer Wasser enthaltenden Mischung, bevorzugt durch schnellen Entzug im Allgemeinen in 1 sec bis 5 h, bevorzugt 5 sec bis 1 h, besonders bevorzugt 10 sec bis 30 min.

Die beschriebenen hochkonzentrierten Formaldehydlösungen werden bevorzugt durch zumindest teilweise Verdampfung der niedriger konzentrierten wässrigen Formaldehydlösungen hergestellt, wobei eine thermische Auftrennung erfolgt. Diese kann einstufig oder mehrstufig, im Gleich- oder im Gegenstrom durchgeführt werden.

Die Verdampfung kann unter Einsatz eines Wendelrohr- bzw. Schlangenrohrverdampfers durchgeführt werden, wobei die Ausgangslösung unter Druck einem Vorwärmer zugeführt, dort aufgeheizt und anschließend unter Dampfbildung entspannt wird. Im beheizten Wendelrohr wird die Lösung dann bis zum Endprodukt aufkonzentriert.

Demgegenüber war es Aufgabe der vorliegenden Erfindung, das Verfahren zur Aufkonzentrierung einer wässrigen Formaldehydlösung durch Verdampfern von Wasser in einem Wendelrohrverdampfer weiter zu verbessern.

Die Lösung besteht in einem Verfahren zur Herstellung einer hochkonzentrierten Formaldehydlösung durch Abtrennung von Wasser aus einer niedriger konzentrierten Formaldehydlösung mit einem Formaldehydgehalt zwischen 5 und 50 Gew.-%, wonach man die niedriger konzentrierte Formaldehydlösung einem Vorwärmer zuführt und im Vorwärmer erhitzt, über eine Druckhaltevorrichtung entspannt und in einem Wendelrohrverdampfer unter Erhalt eines Brüdenstromes sowie der hochkonzentrierten Formaldehydlösung als Sumpfstrom aufkonzentriert, das dadurch gekennzeichnet ist, dass man die erhitzte niedriger konzentrierte Formaldehydlösung in der Druckhaltevorrichtung zu einem zweiphasigen Gemisch entspannt, das man dem Wendelrohrverdampfer zuführt.

Eine entscheidende Verbesserung des Verfahrens zur Aufkonzentrierung im Wendelrohrverdampfer wird somit erfindungsgemäß erreicht, indem man demselben die aufzukonzentrierende wässrige Formaldehydlösung als zweiphasiges Gemisch zuführt.

Das Verfahren der vorliegenden Erfindung geht aus von einer niedriger konzentrierten wässrigen Formaldehydlösung, das heißt einer wässrigen Formaldehydlösung mit einem Gesamtgehalt an Formaldehyd in Form von monomerem Formaldehyd, Methylenglykol und Polyoxymethylenglykolen zwischen 5 und 50 Gew.-%.

Diese Ausgangslösung wird zunächst mit Hilfe eines Vorwärmers aufgeheizt. Als Vorwärmer können herkömmliche Apparatetypen wie Rohrbündelapparate, Plattenwärmeübertrager, Spiralwärmeübertrager oder Elektroerhitzer eingesetzt werden.

Der Druck im Vorwärmer wird durch ein nachgeschaltetes Druckhalteventil vorteilhafter Weise so eingestellt, dass die wässrige Lösung an keiner Stelle im Vorwärmer verdampft. Die im Vorwärmer aufgeheizte Ausgangslösung wird anschließend in einer Druckhaltevorrichtung zu einem zweiphasigen Gemisch entspannt, das heißt zu einer gasförmigen und einer flüssigen Phase. Dieses zweiphasige Gemisch wird dem Wendelrohrverdampfer zugeführt.

Indem das Gemisch zweiphasig zugeführt wird, kommt es zu einer Durchmischung der Phasen im Wendelrohrverdampfer, ohne dass hierfür bewegte Apparateteile erforderlich wären.

Eine weitere Verbesserung der Durchmischung kann durch eine geeignete Auslegung der Geometrie, insbesondere des Rohrdurchmessers des Wendelrohrverdampfers, des Gesamtmengenstromes durch den Apparat sowie des Gasanteils im zweiphasigen Gemisch erreicht werden, wobei bevorzugt ein Strömungsprofil entsprechend einer welligen Filmströmung im Wendelrohrverdampfer erreicht wird.

Dadurch kommt es zu einer intensiven Durchmischung des Flüssigkeitsfilmes, so dass Temperatur- und Konzentrationsgradierenden im Flüssigkeitsfilm wirksam abgebaut werden. Weiterhin liegen hohe Schubspannungen im Wandbereich vor, so dass der Aufbau von Feststoffen an den beheizten Wänden des Apparates wirkungsvoll vermieden wird. Im Allgemeinen werden im Wendelrohrverdampfer Gasgeschwindigkeiten von 20 m/s bis zu mehreren 100 m/s eingestellt.

Durch die geeignete Wahl der Beheizungstemperatur des Apparates wird die zu erreichende Abdampfrate und damit die Konzentration des Formaldehyds im Endprodukt, gesteuert.

Aus dem Wendelrohrverdampfer wird ein Brüdenstrom abgezogen, der einem nachgeschalteten Brüdenabscheider zwecks Auftrennung von Flüssigkeits- und Gasphase zugeführt wird.

Die Gasphase kann in herkömmlichen Kondensatoren, die vorzugsweise als Quenchkondensatoren betrieben werden können, beispielsweise in stehenden Rohrbündelapparaten, vollständig oder partiell kondensiert werden. Die anfallenden Kondensate, die neben Wasser noch Formaldehyd und Methylenglykole enthalten, können in herkömmlichen Verdampfern weiter bis auf ca. 50 Gew.-% Formaldehyd aufkonzentriert werden.

Die so erhaltenen Formaldehydlösungen können vorteilhaft erneut als Zulaufstrom in die Wendelrohrverdampferanlage, speziell in den Vorwärmer, recycliert werden.

Der Gasanteil im zweiphasigen Gemisch, das dem Wendelrohrverdampfer zugeführt wird, kann beispielsweise auch beeinflusst werden, indem man demselben vor der Zuleitung zum Wendelrohrverdampfer ein Strippgas, bevorzugt Stickstoff, zumischt.

Die Verwendung von Stabilisatoren zur Unterdrückung des Feststoffausfalls, die bei chemischen Reaktionen gegebenenfalls stören können, ist im erfindungsgemäßen Verfahren nicht erforderlich. Es ist jedoch möglich, dem zweiphasigen Gemisch vor der Zuführung desselben in den Wendelrohrverdampfer einen Stabilisator zuzusetzen. Die Auswahl des Stabilisators ist hierbei nicht begrenzt. Die Stabilisatoren können vorzugsweise ausgewählt sein aus Methanol, Ethanol, Propanolen, Butanolen, Harnstoff oder Melamin. Zur weiteren Verbesserung der Phasendurchnlischung im Wendelrohrverdampfer ist es vorteilhaft, hierfür geeignete Einrichtungen vorzusehen, insbesondere Ventile, Drosseln, Rippen oder Drahtgestricke.

Neben dem Betrieb im einmaligen Durchgang ist es möglich, den Sumpfstrom aus dem Wendelrohrverdampfer ganz oder teilweise in die Wendelrohrverdampfer-Anlage, das heißt in den Vorwärmer, zu recyclieren.

Es ist auch möglich, die Aufkonzentrierung der wässrigen Formaldehydlösung durch Hintereinanderschaltung von zwei oder mehreren Wendelrohrverdampferanlagen mehrstufig, bevorzugt als Wärmeintegrationsverschaltung, durchzuführen. Hierbei können auch, insbesondere in der oder den ersten Stufen der Aufkonzentrierung herkömmliche Apparate, das heißt von Wendelrohrverdampfern verschiedene Apparate, insbesondere Fallfilmverdampfer eingesetzt werden.

Das erfindungsgemäße Verfahren hat somit den Vorteil, dass durch die spezielle Betriebsweise des Wendelrohrverdampfers sehr hohe flächenspezifische Leistungen bei kurzen Verweilzeiten erreicht werden. Für diese Betriebsweise ist insbesondere maßgeblich, dass der Zulauf zum Wendelrohrverdampfer zweiphasig ist, das heißt einen Gas- und einem Flüssigphasenanteil aufweist. Darüber hinaus kann die Betriebsweise des Wendelrohrverdampfers weiter durch eine geeignete Auslegung der Geometrie desselben, insbesondere des Durchmessers, durch die Festlegung des durchzuleitenden Gesamtmengenstromes sowie des Gasanteiles im zweiphasigen Zulauf verbessert werden.

Durch die kurzen Verweilzeiten infolge der eingestellten, höheren Strömungsgeschwindigkeiten wird die Bildung höherer Polyoxymethylenglykole (entsprechend dem vollständig eingestellten thermodynamischen Gleichgewicht) wirksam verhindert, so dass die aufkonzentrierten Lösungen über vergleichsweise lange Zeiten homogen, das heißt ohne die Bildung fester Phasen, bleiben.

Aufgrund der geringen Verweilzeiten der Lösung bei höheren Temperaturen wird darüber hinaus die unerwünschte Bildung von Ameisensäure aus Formaldehyd wirksam unterdrückt.

Die Erfindung wird im Folgenden anhand eines Ausführungsbeispiels näher erläutert:

Beispiel (zum Vergleich):

Eine wässrige Lösung, enthaltend 50 Gew.-% Formaldehyd, wurde in einem Mengenstrom von 10 kg/h einer Fallfilmverdampferanlage zugeführt. Die Anlage war mit einem Verdampferrohr mit den Maßen 25 x 2 x 3500 mm (Rohraußendurchmesser x Rohrdicke x Rohrlänge), mit einer wärmeübertragenden Fläche von etwa 0,23 m$^2$, ausgestattet. Der Druck im Brüdenraum wurde auf 100 mbar eingestellt, die Beheizungstemperatur auf ca. 135°C.

Es wurde eine Abdampfrate von ca. 17 % erzielt. Damit stellte sich ein Formaldehyd-Gesamtgehalt im Sumpfprodukt von 60 Gew.-% ein.

Die Lösung zeigte bereits im Brüdenraum des Fallfilmverdampfers einen Anfall von festen Oligomeren des Formaldehyds.

Versuche, den Apparat bei höheren Eindarnpfraten zu betreiben, führten zu einem schnellen und irreversiblen Belegen der Heizflächen.

Beispiel (erfindungsgemäß):

Eine wässrige Lösung gleicher Ausgangskonzentration wie im Vergleichsbeispiel, das heißt mit 50 Gew.-% Formaldehyd, wurde in einem Mengenstrom von 15 kg/h einer Wendelrohrverdampferanlage, umfassend als wichtigste Apparate einen Vorwärmer, ein Druckhalteventil und einen Wendelrohrverdampfer, zugeführt. Der Wendelrohrverdampfer war mit einer Glaswendel der Länge 6 m, Innendurchmesser 7 mm und einer wärmeübertragenden Fläche von etwa 0,19 m$^2$, ausgestattet.

Der Druck am Austritt des Vorwärmers wurde auf 1,7 bar, der Druck am Austritt des Wendelrohrverdampfers auf 100 mbar eingestellt.

Die Beheizungstemperatur im Vorwärmer betrug 124°C, im Wendelrohrverdampfer 128°C.

Die Produktaustrittstemperatur am Vorwärmer betrug etwa 103°C, am Austritt des Wendelrohrverdampfers ca. 65°C. Beim Eintritt in den Wendelrohrverdampfer lag der Gasanteil bei 2 % bezogen auf den Gesamtstrom. Infolge der Beheizung und des Druckabfalls entlang des Wendelrohrverdampfers stieg der Gasanteil am Austritt des Wendelrohrverdampfers auf ca. 35 % an.

Im Sumpfprodukt stellte sich ein Formaldehyd-Gesamtgehalt von ca. 75 Gew.-% ein.

Die aufkonzentrierte Lösung war über einen Zeitraum von über 2 h klar, das heißt ohne Ausfall von Feststoffen.

Eine Belagbildung wurde in der Wendelrohrverdampferanlage nicht beobachtet.

# EP 1 723 094 B1

## Patentansprüche

1. Verfahren zur Herstellung einer hochkonzentrierten Formaldehydlösung durch Abtrennung von Wasser aus einer niedriger konzentrierten Formaldehydlösung mit einem Formaldehydgehalt zwischen 5 und 50 Gew.-%, wonach man die niedriger konzentrierte Formaldehydlösung einem Vorwärmer zuführt und im Vorwärmer erhitzt, über eine Druckhaltevorrichtung entspannt und in einem Wendeh-ohrverdampfer unter Erhalt eines Brüdenstromes sowie der hochkonzentrierten Formaldehydlösung als Sumpfstrom aufkonzentriert, **dadurch gekennzeichnet, dass** man die erhitzte niedriger konzentrierte Formaldehydlösung in der Druckhaltevorrichtung zu einem zweiphasigen Gemisch entspannt, das man dem Wendelrohrverdampfer zuführt.

2. Verfahren nach Anspruch 1, **dadurch gekennzeichnet, dass** die hochkonzentrierte Formaldehydlösung im Sumpfstrom des Wendelrohrverdampfers mindestens 70 Gew.-% Formaldehyd, bevorzugt mindestens 75 Gew.-% Formaldehyd, enthält.

3. Verfahren nach Anspruch 1 oder 2, **dadurch gekennzeichnet, dass** man dem zweiphasigen Gemisch vor der Zuführung desselben in den Wendelrohrverdampfer ein Strippgas, bevorzugt Stickstoff, zumischt.

4. Verfahren nach einem der Ansprüche 1 bis 3, **dadurch gekennzeichnet, dass** man dem zweiphasigen Gemisch vor der Zuführung desselben in den Wendelrohrverdampfer einen Stabilisator, insbesondere Methanol, Ethanol, ein Propanol, ein Butanol, Harnstoff oder Melamin, zuführt.

5. Verfahren nach einem der Ansprüche 1 bis 4, **dadurch gekennzeichnet, dass** man im Wendelrohrverdampfer durch die geeignete Wahl der Geometrie desselben sowie der Betriebsbedingungen, insbesondere des Gesamtmengenstromes sowie des Gasgehaltes im zweiphasigen Gemisch, das man durch den Wendelrohrverdampfer leitet, eine wellige Filmströmung einstellt.

6. Verfahren nach einem der Ansprüche 1 bis 5, **dadurch gekennzeichnet, dass** man im Wendelrohrverdampfer Einrichtungen zur intensiven Durchmischung des zweiphasigen Gemisches vorsieht, insbesondere Ventile, Drosseln, Rippen oder Drahtgestricke.

7. Verfahren nach einem der Ansprüche 1 bis 6, **dadurch gekennzeichnet, dass** man den Brüdenstrom aus dem Wendelrohrverdampfer in einem Kondensator, bevorzugt einem Oberflächenkondensator, besonders bevorzugt in einem Quenchkondensator, partiell oder vollständig kondensiert.

8. Verfahren nach Anspruch 7, **dadurch gekennzeichnet, dass** man den kondensierten Anteil des Brüdenstromes in den Vorwärmer recycliert.

9. Verfahren nach einem der Ansprüche 1 bis 8, **dadurch gekennzeichnet, dass** man den Sumpfstrom aus dem Wendelrohrverdampfer ganz oder teilweise in den Vorwärmer recycliert.

## Claims

1. A process for preparing a high-concentration formaldehyde solution by removing water from a lower-concentration formaldehyde solution having a formaldehyde content of from 5 to 50% by weight, in which the lower-concentration formaldehyde solution is fed to a preheater and heated in the preheater, depressurized via a pressure maintenance device and concentrated in a helical tube evaporator to give a vapor stream and the high-concentration formaldehyde solution as bottom stream, wherein the heated lower-concentration formaldehyde solution is depressurized in the pressure maintenance device to give a two-phase mixture which is fed into the helical tube evaporator.

2. The process according to claim 1, wherein the high-concentration formaldehyde solution in the bottom stream of the helical tube evaporator contains at least 70% by weight of formaldehyde, preferably at least 75% by weight of formaldehyde.

3. The process according to claim 1 or 2, wherein a stripping gas, preferably nitrogen, is mixed into the two-phase mixture before it is fed into the helical tube evaporator.

4. The process according to any of claims 1 to 3, wherein a stabilizer, in particular methanol, ethanol, a propanol, a

5

butanol, urea or melamine, is introduced into the two-phase mixture before it is fed into the helical tube evaporator.

5. The process according to any of claims 1 to 4, wherein a wavy film flow is established in the helical tube evaporator by appropriate choice of the geometry of the evaporator and also of the operating conditions, in particular the total mass flow and the gas content of the two-phase mixture which is passed through the helical tube evaporator.

6. The process according to any of claims 1 to 5, wherein devices for achieving intensive mixing of the two-phase mixture, in particular valves, flow restrictors, ribs or knitted wire meshes, are provided in the helical tube evaporator.

7. The process according to any of claims 1 to 6, wherein the vapor stream from the helical tube evaporator is partially or completely condensed in a condenser, preferably a surface condenser, particularly preferably in a quench condenser.

8. The process according to claim 7, wherein the condensed part of the vapor stream is recycled to the preheater.

9. The process according to any of claims 1 to 8, wherein all or part of the bottom stream from the helical tube evaporator is recycled to the preheater.


**Revendications**

1. Procédé pour la préparation d'une solution de formaldéhyde hautement concentrée par séparation d'eau d'une solution de formaldéhyde faiblement concentrée, présentant une teneur en formaldéhyde entre 5 et 50% en poids, selon lequel on introduit la solution de formaldéhyde faiblement concentrée dans un préchauffage et on la chauffe dans le préchauffage, on la détend via un dispositif de maintien en pression et on la concentre dans un évaporateur à tube hélicoïdal avec obtention d'un flux de vapeur ainsi que de la solution de formaldéhyde hautement concentrée comme flux de fond, **caractérisé en ce qu'**on détend la solution de formaldéhyde faiblement concentrée chauffée, dans le dispositif de maintien en pression, en un mélange à deux phases que l'on introduit dans l'évaporateur à tube hélicoïdal.

2. Procédé selon la revendication 1, **caractérisé en ce que** la solution de formaldéhyde hautement concentrée dans le flux de fond de l'évaporateur à tube hélicoïdal contient au moins 70% en poids de formaldéhyde, de préférence au moins 75% en poids de formaldéhyde.

3. Procédé selon la revendication 1 ou 2, **caractérisé en ce qu'**on mélange un gaz d'extraction, de préférence de l'azote, dans le mélange à deux phases, avant son introduction dans l'évaporateur à tube hélicoïdal.

4. Procédé selon l'une quelconque des revendications 1 à 3, **caractérisé en ce qu'**on introduit dans le mélange à deux phases, avant son introduction dans l'évaporateur à tube hélicoïdal, un stabilisateur, en particulier du méthanol, de l'éthanol, un propanol, un butanol, de l'urée ou de la mélamine.

5. Procédé selon l'une quelconque des revendications 1 à 4, **caractérisé en ce qu'**on règle un flux en film ondulé dans l'évaporateur à tube hélicoïdal, par le choix approprié de la géométrie de celui-ci ainsi que des conditions d'exploitation, en particulier du flux de quantités de gaz ainsi que de la teneur en gaz dans le mélange à deux phases que l'on guide au travers de l'évaporateur à tube hélicoïdal.

6. Procédé selon l'une quelconque des revendications 1 à 5, **caractérisé en ce qu'**on prévoit, dans l'évaporateur à tube hélicoïdal, des dispositifs pour un mélange intensif du mélange à deux phases, en particulier des soupapes, des vannes d'étranglement, des ailettes ou des treillis métalliques.

7. Procédé selon l'une quelconque des revendications 1 à 6, **caractérisé en ce qu'**on condense partiellement ou complètement le flux de vapeur provenant de l'évaporateur à tube hélicoïdal dans un condenseur, de préférence un condenseur de surface, de manière particulièrement préférée dans un condenseur instantané.

8. Procédé selon la revendication 7, **caractérisé en ce qu'**on recycle la proportion condensée du flux de vapeur dans le préchauffage.

9. Procédé selon l'une quelconque des revendications 1 à 8, **caractérisé en ce qu'**on recycle le flux de fond provenant de l'évaporateur à tube hélicoïdal complètement ou partiellement dans le préchauffage.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10238248 A

**In der Beschreibung aufgeführte Nicht-Patentliteratur**

- **ULLMANN'2.** Encyclopedia of Industrial Chemistry. 2000